# EUROPEAN PATENT SPECIFICATION

(11) **EP 3 295 172 B1**
(45) Date of publication and mention of the grant of the patent: **02.10.2019**
(21) Application number: 16730487.2
(22) Date of filing: 12.05.2016
(51) Int. Cl.: G01N 33/50, G01N 33/569

(54) **MICROPARTICLES RELEASED BY NATURAL-KILLER CELLS**
NATÜRLICHE KILLERZELLEN FREIGESETZTEN MIKROPARTIKEL
MICROPARTICULES LIBÉRÉES PAR DES CELLULES TUEUSES NATURELLES

(30) Priority: 13.05.2015 IT UB20150463
(43) Date of publication of application: 21.03.2018
(73) Proprietor: Universita' Degli Studi Di Genova, 16126 Genova (IT)
(72) Inventor: DE MARIA, Andrea, I-16124 Genova (IT); BOZZANO, Federica, I-16149 Genova (IT); MARRAS, Francesco, I-16166 Genova (IT)
(74) Representative: Calogero, Ida
(86) International application number: PCT/IB2016/052723
(87) International publication number: WO 2016/181334

(56) References cited:
- US-A1- 2013 295 044
- US-A1- 2014 037 631
- ARIANE ROSEBLADE ET AL: "Cell-derived microparticles: new targets in the therapeutic management of disease", JOURNAL OF PHARMACY & PHARMACEUTICAL SCIENCES : A PUBLICATION OF THE CANADIAN SOCIETY FOR PHARMACEUTICAL SCIENCES, SOCIÉTÉ CANADIENNE DES SCIENCES PHARMACEUTIQUES, vol. 16, no. 2, 1 January 2013 (2013-01-01), page 238, XP055214794, Canada

## Description

The present invention relates to microparticles released by natural killer cells, wherein said microparticles are characterized by the expression of the surface markers NKp30 and NKp46, and by inclusion of perforins and cytokines IFNγ and TNFα, to a method for the identification of said microparticles and the uses of said microparticles as a tool for studying natural killer cells under physiopathological situations.

Natural killer (NK) cells belong to the innate immunity compartment and play an important role in defence from viruses, bacteria, cancer cells.

In human beings, they represent about 5-10% of the lymphocytes circulating in the peripheral blood and are divided into two cell subsets: CD56dim and CD56bright. The two subsets phenotypically differ in the presence/absence on their surfaces, of receptor activators or inhibitors (NKp30, NKp46, NKp44, LIR, KIR).

In literature it can also be read that the two subsets of NK cells show different functions: CD56bright NK cells appear as the main secretors of cytokines/chemokines, whereas CD56dim NK cells mainly have a cytotoxic function (Moretta L. et al., 2004).

Chronic infections from viruses such as HIV, HCV and
tuberculosis can alter the phenotype and function of natural killer cells (De Maria A. et al., 2003; Bozzano F. et al., 2011; Marras F. et al., 2013).

Microparticles (MP) are released by most eukaryote cells and act as long- or short-ray signal vectors, facilitating the exchange of information between donor and recipient cells (Deathrage B.L. et al., 2012; Gyorgy B. et al., 2011; Jaiswal R. et al., 2012; Jaiswal R. et al., 2013).

Roseblade, A. et al. is a review article on cell-derived microparticles, including those from white blood cells, and their role in disease.

Microparticles (MP) are small closed fragments of plasmatic membrane with dimensions ranging from 0.1-1 microns and typically express phosphatidilserine (PS). Analytical approaches for the phenotypical and functional analysis of microparticles comprise Western Blot, flow cytofluorimetry analysis (FACS) or proteomic analysis (Erdbrügger U. et al., 2014).

Literature describes the analysis of microparticles released by different cell populations present in the peripheral blood (T lymphocytes, thymocytes, monocytes, dendrite cells, etc..) but also in other tissues (pancreatic cells, lung cells, liver cancer and not, etc.) and the data in the possession of the scientific community are inserted in a database available online (see web site https://www.microvesicles.org).

The main isolation method of microparticles from biological fluids is differential centrifugation (18,000-20,000 g) (Yuana Y et al., 2010) and among the cell populations releasing microparticles that have been well characterized, platelets, erythrocytes, and endothelial cells can be mentioned.

The main markers analyzed on the surface of microparticles released in biological fluid - whether it be plasma, serum or cell culture supernatant - are Annexin V (PS) and specific cell markers from which microparticles are released, such as, for example CD31, CD41, CD45, CD235a and CD42a (Thery C. et al., 2009; Dignat-George F. et al., 2011; Cocucci E. et al., 2009; Leroyer A.S. et al.; 2010; Aharon A. et al., 2009; Piccin A. et al., 2007; Little K.M. et al., 2010; Amabile N. et al., 2010).

As of today, no microparticles released by natural killer cells have ever been found.

The authors of the present invention have found for the first time, that the two populations of natural killer cells CD56dim and CD56bright are capable of releasing microparticles following stimulation of different origins (cytokine stimuli, chemokine, following a reverse antibody-dependent cytotoxicity in the presence of a target cell line).

Such microparticles are characterized by a specific phenotype profile that allows their identification in biological fluids without having to resort to centrifugation techniques which reduce the microparticles in numerical terms.

In particular, the authors of the present invention have found the presence, on the surface of the microparticles, of markers such as, for example, NKp30 and NKp46 (typical of NK cells), and the characteristic of containing perforins and cytokines, such as, for example, IFNγ and TNFα.

On the basis of this evidence, a method has been developed, which allows the microparticles released on the part of natural killer cells after stimulation to be identified, and to characterize them from a phenotypical and/or functional point of view.

The method for the identification of the microparticles released by natural killer cells in biological fluids (i.e. plasma, serum or culture supernatants) introduces the novelty of the absence of any washing or dilution of the supernatant being analyzed, in order to reduce damage to and the loss, in numerical terms, of the microparticles to the minimum, allowing a high-quality phenotypical and functional analysis. The method also allows the functional analysis of microparticles by evaluation of the content
of cytokines (IFNγ and TNFα) and perforins.

Therefore an object of the present invention relates to microparticles released from natural killer cells, characterized in that they express the surface markers NKp30 and NKp46, and include in their interior perforins and cytokines such as IFNγ and TNFα.

According to preferred embodiments of the invention, the microparticles are released from the populations of natural killer cells CD56dim and CD56bright.

A further object of the present invention relates to a method for the identification of microparticles released by said natural killer cells (preferably by the cell populations CD56dim and CD56bright) comprising the following steps:
a) collecting natural killer cells from a biological sample;
b) stimulating the natural killer cells with PMA and ionomycin in the wells or tubes;
c) adding cytokines and/or a natural killer target cell line and/or monoclonal antibodies directed against the natural cytotoxicity receptors (NCR) of the natural killer cells in the wells or tubes;
d) incubating the cells in the wells or tubes in a culture medium for 4, 16 or 72 hours according to the stimulation of step c);
e) collecting supernatant;
f) intracytoplasmic staining for the evaluation of the content of perforins, IFNγ, TNFα and surface staining of the microparticles with monoclonal antibodies anti-CD56, anti-CD16, anti-NKp30, anti-NKp46 and finally, acquisition using a cytofluorimeter.

According to the present invention, the biological sample can be peripheral blood of a human being (serum or plasma, in particular) or a cell culture supernatant. The individual can be healthy or pathological, adult or paediatric.

The wells in which the various types of stimulation of the NK cells, including the maximal with PMA and Ionomycin, are to be effected, are preferably wells of 24-well flat-bottom plates or 96-well U-shaped plates. The tubes are preferably tubes with a U-shaped bottom.

The cytokines used in step c) for stimulating the natural killer cells, are selected from the group consisting of IL2, IL15, IL12 and mixtures thereof.

The target cell line of natural killer cells is preferably a line of murine mastocytoma P815 FcgR positive (ATCC® TIB-64™).

The monoclonal antibodies directed against the natural cytotoxicity receptors (NCR) of the natural killer cells used in the stimulation step c) are monoclonal antibodies anti-NKp46 (IgG1, clone BAB281) and monoclonal antibodies anti NKp30 (IgG1, clone 7A6).

The natural killer cells isolated from the mononuclear cells of the peripheral blood by immunomagnetic separation are incubated in a suitable 10% culture medium, simple or with the addition of human recombinant cytokines such as IL2, IL12, IL15, or mixtures of IL12 + IL15, for 48 hours or, alternatively, the cells are incubated in the co-presence of the target cell line P815 and monoclonal antibodies directed against the natural cytotoxicity receptors (NCR) of the natural killer cells used in the step for a time equal to 4 hours. The cell line K562, used for testing the functional capacity of the MPs, released in the supernatant of the natural killer cell cultures, is incubated in the presence of the above-mentioned supernatant in a 10% medium for a time equal to 4 hours.

After the period of time defined according to the stimulation chosen, the supernatant is collected taking care not to collect the cells deposited on the bottom of the well (tube).

The intracytoplasmic staining of step f) for the
analysis of the content and surface of the microparticles of interest, is advantageously carried out without any intermediate washing.

The microparticles identified according to the method above described are also part of the present disclosure.

A further object of the present invention relates to the use of the microparticles released by the populations of natural killer cells (preferably the populations of NK cells CD56dim and CD56bright) above defined, as a marker for controlling viral, fungal, bacterial infections or for controlling tumour cells. Thanks to the important role of NK cells in controlling viral infections and tumour cells, in fact, their detection and quantification in:
a) supernatants of in vitro NK cell cultures from any donor (healthy or from patients suffering from neoplasms, chronic infections, autoimmunity);
b) plasma or serum from any donor (healthy or from patients suffering from neoplasms, chronic infections, autoimmunity) and
c) supernatant of primary tumours,
   allows them to be used in establishing prognosis, evolution and the best possible therapies. In particular, said infections can be due to pathogenic agents such as for example HIV, HCV and tuberculosis (TB).

According to a further aspect, the present invention envisages the use of the microparticles released by the populations of natural killer cells CD56dim and CD56bright as a tool for studying the behaviour of natural killer cells in terms of the release of microparticles in physiological and/or pathological situations. The microparticles can be advantageously used, for example, as a marker for monitoring acute or chronic viral infections and in the control of tumour cells.

Finally, after the separation and concentration of the microparticles deriving from NK cells, they can be used as a biological drug for infusion in a patient, above all in locations where the passage of standard pharmacological treatment is difficult.

A further aspect of the disclosure therefore relates to a kit for identifying the microparticles released by natural killer cells comprising the following components:
A) carboxyfluorescein succinimidyl ester dye (CFSE);
B) anti-perforin monoclonal antibodies directly conjugated with fluorochrome;
C) anti-IFNy monoclonal antibodies directly conjugated with fluorochrome;
D) mixture of anti-CD56, anti-CD16, anti-NKp30, anti-NKp46 monoclonal antibodies directly conjugated with fluorochrome;
E) TOPRO3 dye, or any other dye of nucleic acids that does not emit, once excited by the appropriate laser, within the wavelength of phycoerythrin.

The fluorochromes are preferably selected from PeCy7, APC, FITC, phycoerythrin (PE), AlexaFluor647, Horizon V450, BV510, BV650, PerCPCy5.5, PeCy5 and APCH7.

The kit can optionally comprise two sterile 5 ml polystyrene tubes or test-tubes and an illustrative leaflet of the method.

The present description is now described for illustrative but non-limiting purposes, according to a preferred embodiment with particular reference to the enclosed figures, in which:
- Figure 1 shows the phenotypic analysis and content in terms of IFNγ and Perforins of the microparticles released by the natural killer cells isolated from the peripheral blood of a healthy donor;
- Figure 2 shows the functional analysis of the lytic capacity of the microparticles released by natural killer cells with respect to a cell line of human chronic myeloid leukemia such as K562, an elite target of NK cells.

In the following experimental section, the invention is detailed with preferred examples.

### EXAMPLE 1: Identification and phenotypic analysis of the microparticles released by natural killer cells

### MATERIALS AND METHODS

### Biological sample

20 ml of peripheral blood were collected by venipuncture from a healthy donor and diluted with a further 20 ml of RPMI (Lonza/BioWhittaker).

### PROCEDURES

### Separation of peripheral blood mononuclear cells (PBMC)

Two sterile 50 ml polystyrene tubes were prepared into which 10 ml of Ficoll-Hypaque were poured.

The peripheral blood was diluted with 10% medium in a ratio of 1:2 and stratified in the 50 ml tube containing Ficoll.

A centrifugation was effected at 2,000 rpm for 20 minutes at room temperature and without braking in order to have a gradient centrifugation.

A culture medium consisting of 500 ml of RPMI was prepared, to which 10% of fetal calf serum was added, together with 2 mM L-glutamine, and a mixture of antibiotics penicillin and streptomycin 1%, which is then filtered through coffee filters with 0.22 µm pores (Millipore), briefly referred to herein as 10% medium.

At the end of the centrifugation, the central ring between the Ficoll phases containing PBMCs was collected in a sterile 15 ml U-bottom tube with a 5 ml pipette.

The volume was brought to 15 ml with RPMI and the whole mixture was centrifuged at 1100 rpm for 6 minutes at 4°C.

The supernatant was emptied and the pellet of cells was re-suspended in 15 ml of culture medium which was centrifuged at 1,500 rpm for 5 minutes at 4°C.

The supernatant was emptied and the PBMC pellet was brought to a volume of 10 ml with culture medium.

The cells were counted withdrawing 10 µl of PBMC and adding them to 90 µl of Tripan Blue (Sigma) previously diluted 1:5. 10 µl were withdrawn and put in the counting chamber. The cells were counted and the PBMCs were brought to a concentration of 10 million/ml.

### Separation of natural killer (NK) cells from PBMCs

10 million PBMC cells (1 ml) were transferred to a 15 ml U-bottom tube.

9 ml of buffer were added for the separation, composed of: 74.6% of PBS, 25% of BSA (Bovine Albumin) diluted to 2% and 0.4% of EDTA 0.5 M pH 8.

A centrifugation was effected at 1600 rpm for 5 minutes at 5°C. At the end of the centrifugation, the supernatant is emptied and 10 µl of the mixture of antibodies present in the NK kit Isolation KIT (Miltenyi) are added, following the indications present in the instruction leaflet.

The procedure is briefly as follows:
1) Incubate in ice for 30 minutes
2) Add 20 µl of the mixture of beads
3) Incubate in ice for 30 minutes
4) Add 9 ml of buffer for the separation
5) Centrifuge at 1600 rpm for 5 minutes at 5°C
6) Empty the supernatant and add 500 µl of buffer for the separation
7) Put the column for the separation of the microparticles on the relative magnetic support of the kit
8) Position a sterile 5 ml U-bottom tube beneath the column
9) Activate the separation column by passing 1 ml of buffer through the same for separation
10) Elute the column with 500 µl of suspension of PBMC cells previously prepared
11) Wash the column three times with 1 ml at a time of buffer for the separation
12) Collect the NK cells in the U-tube positioned beneath the column
13) Bring the U-tube to the maximum volume of 5 ml and close with a specific release cap
14) Centrifuge at 1600 rpm for 5 minutes at 5°C
15) Empty the supernatant and re-suspend the NK cells in a 10% medium.

### Stimulation of NK cells after immunomagnetic separation

Various stimulation methods of the natural killer cells are adopted:
i) natural killer cells in 10% medium alone
ii) natural killer cells in 10% medium with the addition of human recombinant cytokine IL2 at a final concentration of 200 U/ml (Peprotech) alone
iii) natural killer cells in 10% medium with the addition of human recombinant cytokine IL12 at a final concentration of 20 ng/ml (Peprotech) alone
iv) natural killer cells in 10% medium with the addition of human recombinant cytokine IL15 at a final concentration of 20 ng/ml (Peprotech) alone
v) natural killer cells in 10% medium with the addition of human recombinant cytokine IL12 at a final concentration of 20 ng/ml (Peprotech) and human recombinant cytokine IL15 at a final concentration of 20 ng/ml (Peprotech) alone
vi) natural killer cells in 10% medium + target cell line of murine mastocytoma P815 (ATCC® TIB-64™)
vii) natural killer cells in 10% medium + target cell line of murine mastocytoma P815 (ATCC® TIB-64™)+ mouse anti-human NKp46 monoclonal antibody + mouse anti-human NKp30 monoclonal antibody
viii) natural killer cells in 10% medium + target cell line P815 + PMA (phorbol 12-myristate 13-acetate, SIGMA Aldrich) at a final concentration of 25 ng/ml (nanogram/ml) + Ionomycin (SIGMA Aldrich) at a final concentration of 1 ug/ml (microgram/ml).

The NK cells were seeded in 24-well plates or in single 5 ml sterile tubes at a concentration of 2,000,000/ml in a final volume equal to 1 ml of 10% medium with or without the addition of human recombinant cytokines at a final concentration of 20 ng/ml.

The following are added to the wells or single tubes designated for this stimulation:
- the target line of murine mastocytoma P815 in a number of 20,000 cells at a concentration of 2,000,000/ml, and
- the monoclonal antibodies anti-NKp46 (IgG1), clone BAB281) and anti NKp30 (IgG1, clone 7A6) at a concentration of 0.1 ug/ml

Incubation is then effected for 16 hours at 37°C, 5% CO₂.

### Analysis of the microparticles present in the supernatant of the natural killer cell cultures

3 disposable sterile U-bottom polystyrene tubes are identified as follows for each individual stimulation for the phenotypic analysis:
a) Blank
b) CFSE (carboxyfluorescein succinimidyl ester dye which binds with the cytoplasmic proteins)
c) CFSE-CD56-CD16-NKp30-NKp46

5 disposable sterile U-bottom polystyrene tubes are identified as follows for each individual stimulation for the analysis of the content of cytokines and perforins:
d) Blank
e) CFSE
f) CFSE-CD56-CD16-NKp30-NKp46-IFNγ
g) CFSE-CD56-CD16-NKp30-NKp46-TNFα
h) CFSE-PERFORINE-CD56-CD16-NKP30-NKP46

A centrifugation is effected of the plate with the wells destined for the stimulations or single tubes, at 1,500 rpm for 5 minutes at 4°C.

500 µl of supernatant are recovered with a 200 µl pipette, transferring them to 2 ml sterile test-tubes, taking care not to also remove the pellet of cells present after the centrifugation on the bottom of the well or single tube.

All the necessary material is therefore recovered:
- supernatant of the NK cells after a culture of 48 hours
- 2.5 µl of monoclonal antibodies anti-CD56 directly conjugated with PeCy7 (Beckman Coulter, see illustrative leaflets) for each tube; 2 µl of monoclonal antibodies anti-CD16 directly conjugated in APC-H7 or FITC or phycoerythrin (PE) (BD Biosciences, see illustrative leaflets) for each tube; 5 µl of monoclonal antibodies anti-NKp30 conjugated with AlexaFluor647 and anti-NKp46 conjugated with Horizon V450 (BD Biosciences, see illustrative leaflets); 2 µl of monoclonal antibodies anti-Perforins conjugated with phycoerythrin (PE) (BD Biosciences, see illustrative leaflets), 5 µl of monoclonal antibodies anti-IFNy conjugated with APC (BD Biosciences, see illustrative leaflets), 10 µl of monoclonal antibodies anti-TNFa conjugated with phycoerythrin (PE; BD Biosciences, see illustrative leaflets)

- CFSE (carboxyfluorescein succinimidyl ester, SIGMA Aldrich) at a final concentration of 5 µM
- permeabilizing/fixing solution Cytofix/Cytoperm (BD Biosciences)
- formaldehyde at 35% diluted to a final concentration at 2% with PBS which is indicated hereunder as Formaldehyde at 2%.

50 µl of plasma of the donor obtained are distributed in the U-bottom tubes from a) to h) previously identified.

Carboxyfluorescein succinimidyl ester (CFSE) is added at a final concentration of 5 µM to the tubes from a) to h).

Incubation is effected for 90 minutes at 4°C away from the light.

The antibodies indicated on each tube are added to the tubes from a)-c), in the quantity previously indicated, whereas 20 µl of Cytofix/Cytoperm (BD Biosciences) are added to the tubes from d)-h).

The tubes are incubated for 20 minutes at 4°C.

At this point, 200 µl of Formaldehyde 2% (in PBS) are added to tubes a)-c), whereas the antibodies indicated on each tube are added to tubes d) to h).

Tubes d)-h) are incubated for 20 minutes at 4°C.

The tubes are acquired by means of a FACSFortessa cytofluorimeter (Becton Dickinson), and the microparticles released by natural killer cells are identified by means of Forward (FSC) and Side Scatter (SSC) physical parameters, acquiring 10,000 events in the physical gate FSC/SSC and CFSE positive.

The data acquired with FlowJo (TreeStar) software were then analyzed.

### Sorting of the microparticles from cell supernatant by means of a cytofluorimeter

The same procedure as that described above is basically adopted.

The signal is acquired with a FACSAria (Becton Dickinson) and the microparticles released by natural killer cells are identified by means of Forward (FSC) and Side Scatter (SSC) physical parameters, acquiring 10,000 events in the physical gate FSC/SSC and CFSE positive.

### RESULTS

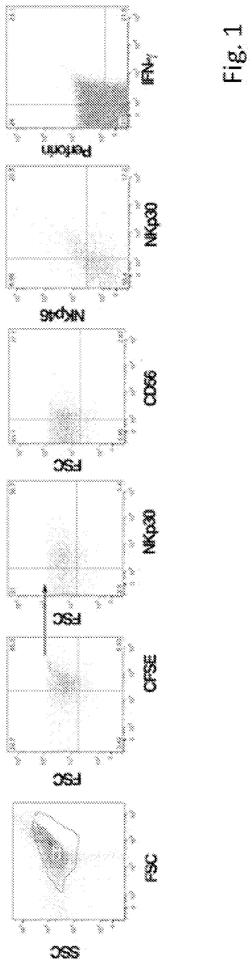

Figure 1 shows the results of the phenotypic analysis of the microparticles released by natural killer cells isolated from peripheral blood of a healthy donor coming from the Blood Bank of the Gaslini Hospital in Genoa, after informed consent.

The microparticles are obtained by immunomagnetic separation and stimulated as previously described with 10% medium containing human recombinant cytokine IL2 at a final concentration of 200 U/ml for 48 hours.

The study of the markers CD56, NKp30 and NKp46 was carried out using the cytofluorimetric technique.

The microparticles released in the supernatant were physically identified by FSC/SSC and as positivity for the CFSE dye that remains inside the membrane of the microparticles.

Taking into consideration the microparticles CFSE positive (+) alone, and therefore with the membrane integral, the analysis of the classical markers of natural killer cells shows that 36.7% proves to be NKp30 positive, 27% CD56 positive whereas 20.5% of the MPS proves to co-express NCR NKp46 and NKp30.

The last dot plot shows how IFNγ, perforins are contained in the CFSE+ microparticles, and in 23.1% both IFNγ and perforins are present.

### EXAMPLE 2: Functional analysis of the microparticles released by natural killer cells

The analysis of the function of the microparticles deriving from the supernatant of the natural killer cell cultures post-stimulation, was carried out according to the following protocol.

### MATERIALS AND METHODS

2,000,000 erythroleukemia cells are recovered of the cell line of human chronic myelogenous leukemia K562 (ATCC® CCL-243™) which represents the election target for natural killer cells, at a concentration of 20,000/ml.

The supernatant of the natural killer cell cultures after the single stimulations described in Example 1, was used.

A culture medium consisting of 500 ml of RPMI is used, to which 10% of fetal calf serum was added, together with 2 mM L-glutamine, and a mixture of antibiotics penicillin and streptomycin 1%, which is then filtered through coffee filters with 0.22 µm pores (Millipore) briefly referred to herein as 10% medium.

The dyes/markers used are PKH26 (Sigma Aldrich) and TOPRO3 (Invitrogen), Cytofix/cytoperm (BD Biosciences) is used as fixing/permeabilizing solution.

The following single sterile 5 ml polystyrene tubes (or alternatively 24-well plate wells) are prepared and identified:
- cell line K562 alone without PKH26
- cell line K562 marked with PKH26
- cell line K562 marked with PKH26 + stimulation supernatant of natural killer cells in 10% medium
- cell line K562 marked with PKH26 + stimulation supernatant of natural killer cells in 10% medium + IL2
- cell line K562 marked with PKH26 + stimulation supernatant of natural killer cells in 10% medium + IL15
- cell line K562 marked with PKH26 + stimulation supernatant of natural killer cells in 10% medium + IL15 + IL12
- cell line K562 marked with PKH26 + stimulation supernatant of natural killer cells in 10% medium + cell line of murine mastocytoma P815 + mAb anti-NKp46 + mAb anti-NKp30
- cell line K562 + Cytofix/Cyperm.

### PROCEDURE

1,000,000 cells of human chronic myelogenous leukemia K562 are introduced at a concentration of 2,000,000/ml in diluent C (ready solution present in the Kit PKH26, Sigma Aldrich), into a sterile 5 ml U-bottom polystyrene tube.

The dilution of PKH26 (Sigma Aldrich) is prepared as indicated in the illustrative leaflet, as follows:
500 µl of diluent C (ready solution present in the Kit PKH26, Sigma Aldrich) are introduced into a sterile 5 nml polystyrene tube and 2.5 µl of the mother solution of PKH26 are added.

The solution of diluted PKH26 is added to the tube containing the cell line K562 prepared as described above.

The tube is incubated at room temperature for 5 minutes.

The whole mixture is brought to a volume of 5 ml by adding 4.5 ml of 10% medium.

Centrifugation is then effected at 1,500 rpm at 4°C for 5 minutes.

The supernatant is emptied and the K562 cells are brought back to a concentration of 2,000,000/ml in 10% medium.

20,000 K562 cells marked with PKH26 and 20,000 K562 cells not marked are introduced into the tubes as previously listed in the section materials and methods.

50 µl of each supernatant are added to the respective tubes.

Incubation is effected at 37°C, 5% CO₂ for 4 hours.

The dye TOPRO3 is diluted (monomer of carbocyanine, even if any other dye of nucleic acids could be used, that does not emit, once excited by the appropriate laser within the wavelength of phycoerythrin, the emission length of the dye PKH26) in PBS 1:100 and 20 µl are added to each tube only before acquiring the single tube with a cytofluorimeter.

The tubes are acquired by means of a FACSFortessa cytofluorimeter (Becton Dickinson), and the K562 cells are identified by means of Forward (FSC) and Side Scatter (SSC) physical parameters, acquiring 10,000 events in the physical gate FSC/SSC and PKH26 positive.

### RESULTS

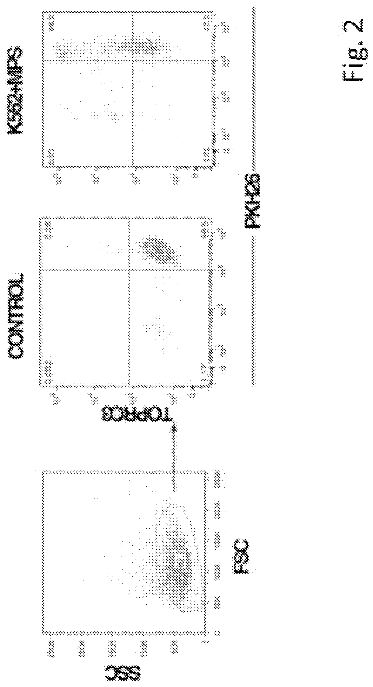

Figure 2 shows the results of the functional analysis of the microparticles released by natural killer cells in terms of lytic capacity with respect to the cell line of human chronic myeloid leukemia K5 62, which represents an elite target of NK cells. This demonstrates the potentiality of the invention of studying the functional properties of the microparticles released by NK cells.

The cells of the target line K562 were identified using a cytofluorimeter for their physical features.

In the control tube, where cells of the target line K562 are exclusively present, they appear marked with K562 and 98.5% negative for the dye TOPRO3 which indicates the cell mortality for which the K562 cells present in the experiment prove to be living and well-marked with PKH26.

The sample tube contains K562 PHK26+ and microparticles deriving from NK cell cultures of a healthy donor and cultivated in 10% medium to which cytokine IL2 has been added. The presence of the microparticles causes a mortality of 50% of the target line as illustrated in the upper right quadrant, where PHK26+TOPRO3+ cells are present.

### BIBLIOGRAPHY

- Moretta L. Blood 2010; 116:3689-91.
- De Maria A. et al. Eur. J. Immunol. 2003; 33 (9): 2410-8.
- Bozzano F. et al. Eur. J. Immunol. 2011; 41 (10): 2905-14.
- Marras F. et al. Proc. Natl. Acad. Sci. USA. 2013; 110 (29): 11970-5.
- Deatherage B.L. et al. Infect. Immun. 2012; 80 (6): 1948-57.
- György B., et al. Cell. Mol. Life Sci. 2011; 68 (16): 2667-88.
- Jaiswal R. et al. Mol Cancer. 2012; 11: 37.
- Jaiswal R. et al. PLoS One. 2013; 8(4): e61515.
- Erdbrügger U. Cytometry A. 2014; 85 (9): 756-70.
- Yuana Y. et al. Thromb. Haemost. 2011; 105 (3): 396-408.
- Théry C. et al. Nat. Rev. Immunol. 2009; 9 (8): 581-93.
- Dignat-George F. et al. Arterioscler. Thromb. Vasc. Biol. 2011; 31 (1): 27-33.
- Cocucci E. et al. Trends Cell. Biol. 2009; 19 (2):43-51.
- Leroyer A.S. et al. Thromb. Haemost. 2010; 104 (3): 456-63.
- Aharon A. et al. Best Pract. Res. Clin. Haematol. 2009; 22 (1): 61-9.
- Piccin A. et al. Blood Rev. 2007; 21(3): 157-71.
- Little K.M. et al. Semin. Thromb. Hemost. 2010; 36 (8): 845-56.
- Amabile N. et al. Semin. Thromb. Hemost. 2010; 36 (8): 907-16.
- Roseblade A. et al. J. Pharm. Pharmaceut. Sci 2013; 16 (2): 238-253.

## Claims

1. Microparticles released by natural killer cells, wherein said microparticles are **characterized by** the expression of the surface markers NKp30 and NKp46, and by inclusion of perforins and cytokines IFNγ and TNFα.

2. Microparticles according to claim 1, that are released by the populations of natural killer cells CD56dim and CD56bright.

3. Method for the identification of the microparticles released by the natural killer cells according to claim 1 comprising the following steps:
a) collecting the natural killer cells from a biological sample;
b) stimulating the natural killer cells with PMA and ionomycin in the wells or tubes;
c) adding cytokines and/or a natural killer target cell line and/or fluorochrome conjugated monoclonal antibodies directed against the natural cytotoxicity receptors (NCR) to the wells or tubes;
d) incubating the cells in the wells or tubes in a culture medium for 4, 16 or 72 hours according to the stimulation of step c);
e) collecting supernatant;
f) intracytoplasmic staining for the evaluation of the content of perforins, IFNγ, TNFα and surface staining of the microparticles with monoclonal antibodies anti-CD56, anti-CD16, anti-NKp30, anti-NKp46 and acquisition by cytofluorimeter.

4. Method according to claim 3, wherein said natural killer cells belong to the populations CD56dim and CD56bright.

5. Method according to anyone of the claims 3-4, wherein the biological sample is selected between peripheral blood, serum or plasma of a subject or a supernatant of a cell culture.

6. Method according to anyone of the claims 3-5, wherein said subject is an healthy or pathological subject, adult or paediatric.

7. Method according to anyone of the claims 3-6, wherein said wells belong to 24-well flat bottom plates or 96-well U-shape plates and said tubes are preferably U-shaped tubes.

8. Method according to anyone of the claims 3-7, wherein the cytokines used in step c) of stimulation of the natural killer cells are IL2, IL15, IL12.

9. Method according to anyone of the claims 3-8, wherein the natural killer target cell line used in step c) of stimulation is the murine mastocytoma cell line P815.

10. Method according to anyone of the claims 3-9, wherein the monoclonal antibodies directed against the natural cytotoxicity receptors of the natural killer cells used in step c) of stimulation are monoclonal antibodies anti-NKp46 and monoclonal antibodies anti-NKp30 conjugated with fluorochrome.

11. Method according to claim 10, wherein said fluorochrome is selected from the group consisting of AlexaFluor647, Horizon V450, PeCy7, APC, FITC, PE, BV510, BV650, PerCPCy5.5, PeCy5 and APCH7.

12. Use of the microparticles according to anyone of the claims 1-2, as a marker for ex-vivo controlling viral, fungal, bacterial infections or for ex-vivo controlling tumoral cells.

13. Use of the microparticles according to anyone of the claims 1-2, as a tool for ex-vivo studying the behaviour of the natural killer cells in physiologic and/or pathologic state.

## Patentansprüche

1. Von natürlichen Killerzellen freigesetzte Mikropartikel, wobei die Mikropartikel **gekennzeichnet sind durch** die Expression der Oberflächenmarker NKp30 und NKp46 und durch die Inklusion von Perforinen und Cytokinen IFNγ und TNFα.

2. Mikropartikel nach Anspruch 1, die von den Populationen natürlicher Killerzellen CD56dim und CD56bright freigesetzt werden.

3. Verfahren zur Identifizierung von Mikropartikel, die durch natürliche Killerzellen nach Anspruch 1, freigesetzt werden, umfassend folgende Schritte:
a) Sammeln der natürlichen Killerzellen aus einer biologischen Probe;
b) Stimulation der natürlichen Killerzellen mit PMA und Ionomycin in den Mulden oder Röhren;
c) Hinzufügen von Zytokinen und/oder natürlichen Killer-Target-Zelllinien und/oder fluorochrom-konjugierte monoklonale Antikörper gegen die natürlichen Zytotoxizitätsrezeptoren (NCR) in die Mulden oder Röhren;
d) Inkubieren der Zellen in den Mulden oder Röhren in einem Kulturmedium für 4, 16 oder 72 Stunden entsprechend der Stimulation in Schritt c);
e) Sammeln des Überstandes;
f) intrazytoplasmisches Färben zur Bewertung des Gehalts an Perforinen, IFNγ, TNFα und Färben der Mikropartikeloberflächen mit monoklonalen Antikörpern Anti-CD56, Anti-CD16, Anti-NKp30, Anti-NKp46 und Akquisition durch Zytofluorimeter.

4. Verfahren nach Anspruch 3, wobei die natürlichen Killerzellen zu den Populationen CD56dim und CD56bright gehören.

5. Verfahren nach einem der Ansprüche 3-4, wobei die biologische Probe ausgewählt ist aus peripherem Blut, Serum oder Plasma einer Person oder einem Überstand aus einer Zellkultur.

6. Verfahren nach einem der Ansprüche 3-5, wobei die Person eine gesunde oder eine kranke Person im Erwachsenen- oder Kindesalter ist.

7. Verfahren nach einem der Ansprüche 3-6, wobei die Mulden zu 24-Mulden-Flachbodenplatten oder 96-Mulden-U-Form-Platten gehören und die Röhren vorzugsweise U-förmige Röhren sind.

8. Verfahren nach einem der Ansprüche 3-7, wobei die in Schritt c) verwendeten Cytokine zur Stimulation der natürlichen Killerzellen IL2, IL15, IL12 sind.

9. Verfahren nach einem der Ansprüche 3-8, wobei die in Schritt c) verwendeten Cytokine zur Stimulation der natürlichen Killerzellen IL2, IL15, P815 sind.

10. Verfahren nach einem der Ansprüche 3-9, wobei die monoklonalen Antikörper, die gegen die natürliche Zytotoxizitätsrezeptoren der in Schritt c) zur Stimulation verwendeten natürlichen Killerzellen monoklonale Antikörper Anti-NKp46 und monoklonale Antikörper Anti-NKp30 sind, die mit Fluorochrom konjugiert sind.

11. Verfahren nach Anspruch 10, wobei das Fluorochrom ausgewählt aus der Gruppe bestehend aus AlexaFluor647, Horizon V450, PeCy7, APC, FITC, PE, BV510, BV650, PerCPCy5.5, PeCy5 und APCH7.

12. Verwendung der Mikropartikel nach einem der Ansprüche 1-2 als ein Marker zur Ex-Vivo-Kontrolle viraler, fungaler, baktiereller Infektionen oder zur Ex-Vivo-Kontrolle tumoraler Zellen.

13. Verwendung von Mikropartikeln nach einem der Ansprüche 1-2 als ein Instrument für Ex-Vivo-Studien des Verhaltens der natürlichen Killerzellen in physiologischem und/oder pathologischem Zustand.

## Revendications

1. Microparticules libérées par des cellules tueuses naturelles, dans lesquelles lesdites microparticules sont **caractérisées par** l'expression des marqueurs de surface NKp30 et NKp46, et par l'inclusion des perforines et des cytokines IFNγ et TNFα.

2. Microparticules selon la revendication 1, qui sont libérées par les populations de cellules tueuses naturelles CD56dim et CD56bright.

3. Procédé d'identification des microparticules libérées par les cellules tueuses naturelles selon la revendication 1, comprenant les étapes suivantes :
a) collecter les cellules tueuses naturelles à partir d'un échantillon biologique ;
b) stimuler les cellules tueuses naturelles avec du PMA et de l'ionomycine dans les puits ou les tubes ;
c) ajouter des cytokines et/ou une lignée cellulaire cible tueuse naturelle et/ou des anticorps monoclonaux conjugués au fluorochrome, dirigés contre les récepteurs de cytotoxicité naturelle (NCR) vers les puits ou les tubes ;
d) incuber les cellules dans les puits ou les tubes dans un milieu de culture pendant 4, 16 ou 72 heures selon la stimulation à l'étape c) ;
e) collecter du surnageant ;
f) réaliser la coloration intra-cytoplasmique pour l'évaluation du contenu de perforines, IFNγ, TNFα, la coloration en surface des microparticules avec des anticorps monoclonaux anti-CD56, anti-CD16, anti-NKp30 et anti-NKp46, et l'acquisition par cytofluorimètre.

4. Procédé selon la revendication 3, dans lequel lesdites cellules tueuses naturelles appartiennent aux populations CD56dim et CD56bright.

5. Procédé selon l'une quelconque des revendications 3 à 4, dans lequel l'échantillon biologique est choisi entre le sang périphérique, le sérum ou le plasma d'un sujet ou le surnageant d'une culture cellulaire.

6. Procédé selon l'une quelconque des revendications 3 à 5, dans lequel ledit sujet est un sujet en santé ou en état pathologique, adulte ou pédiatrique.

7. Procédé selon l'une quelconque des revendications 3 à 6, dans lequel lesdits puits appartiennent à des plaques à 24 puits à fond plat ou à des plaques à 96 puits en forme de U et lesdits tubes sont de préférence des tubes en forme de U.

8. Procédé selon l'une quelconque des revendications 3 à 7, dans lequel les cytokines utilisées à l'étape c) de stimulation des cellules tueuses naturelles sont IL2, IL15, IL12.

9. Procédé selon l'une quelconque des revendications 3 à 8, dans lequel la lignée cellulaire cible tueuse naturelle utilisée à l'étape c) de stimulation est la lignée cellulaire de mastocytome murine P815.

10. Procédé selon l'une quelconque des revendications 3 à 9, dans lequel les anticorps monoclonaux dirigés contre les récepteurs de cytotoxicité naturelle des cellules tueuses naturelles utilisées à l'étape c) de stimulation sont des anticorps monoclonaux anti-NKp46 et des anticorps monoclonaux anti-NKp30 conjugués au fluorochrome.

11. Procédé selon la revendication 10, dans lequel ledit fluorochrome est choisi dans le groupe comprenant AlexaFluor647, Horizon V450, PeCy7, APC, FITC, PE, BV510, BV650, PerCPCy5.5, PeCy5 et APCH7.

12. Utilisation des microparticules selon l'une quelconque des revendications 1 à 2, comme marqueur pour le contrôle ex-vivo d'infections virales, fongiques, bactériennes ou pour le contrôle ex-vivo des cellules tumorales.

13. Utilisation des microparticules selon l'une quelconque des revendications 1 à 2, comme outil d'étude ex-vivo du comportement des cellules tueuses naturelles en état physiologique et/ou pathologique.
